(19) Europäisches Patentamt
European Patent Office
Office européen des brevets

(11) **EP 4 691 473 A1**

(12) **EUROPEAN PATENT APPLICATION**
published in accordance with Art. 153(4) EPC

(43) Date of publication:
**11.02.2026 Bulletin 2026/07**

(21) Application number: 23932747.1

(22) Date of filing: **27.10.2023**

(51) International Patent Classification (IPC):
*A61K 31/7048* (2006.01)    *A61K 31/352* (2006.01)
*A61K 31/365* (2006.01)    *A61K 31/435* (2006.01)
*A61K 47/08* (2006.01)    *A61K 47/18* (2017.01)
*A61K 36/11* (2006.01)    *A61P 17/14* (2006.01)

(52) Cooperative Patent Classification (CPC):
Y02A 50/30

(86) International application number:
**PCT/CN2023/127216**

(87) International publication number:
**WO 2024/212476 (17.10.2024 Gazette 2024/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:  **14.04.2023  CN 202310403390**

(71) Applicants:
• **Beijing Academy of TCM Beauty Supplements Co., Ltd.**
**Beijing 102400 (CN)**

• **Nutri-Woods Bio-Tech (Beijing) Co., Ltd.**
**Beijing 101200 (CN)**

(72) Inventors:
• **DU, Yijie**
**Beijing 102400 (CN)**
• **ZHANG, Weihong**
**Beijing 102400 (CN)**
• **ZHAO, Siqi**
**Beijing 102400 (CN)**

(74) Representative: **Breitreiner, Moritz**
**GSKH Patent- und Rechtsanwälte**
**Nymphenburger Straße 14**
**80335 München (DE)**

(54) **PLATYCLADUS ORIENTALIS EXTRACT, PREPARATION METHOD THEREFOR AND USE THEREOF**

(57)    The present disclosure relates to the technical field of cosmetics, and in particular to a Platycladus orientalis extract, a preparation method therefor and use thereof. Also provided is a Platycladus orientalis extract, comprising the following components in parts by mass: 0.121-0.902 parts of isoquercetin, 1.113-2.420 parts of afzelin, 2.825-4.481 parts of amentoflavone, 0.090-2.234 parts of pinusolide, and 510.61-759.44 parts of D-(-)-Quinic acid. The present disclosure provides a Platycladus orientalis extract comprising a certain amount of isoquercetin, afzelin, amentoflavone, pinusolide and D-(-)-Quinic acid, which has the efficacy of preventing and treating alopecia and can be used as an alopecia-preventing raw material in alopecia-preventing products.

$$y = -0.167x^2 + 10.251x + 13.608$$
$$R^2 = 0.99$$

**FIG. 2**

## Description

### CROSS-REFERENCE TO RELATED APPLICATIONS

The present application claims priority to a Chinese patent application with application number of 202310403390.6 filed with the Patent Office of China on April 14, 2023, entitled "PLATYCLADUS ORIENTALIS EXTRACT, PREPARATION METHOD THEREFOR AND USE THEREOF", the entire contents of which are incorporated herein by reference.

### TECHNICAL FIELD

The present disclosure relates to the technical field of cosmetics, and in particular a Platycladus orientalis extract, a preparation method therefor and use thereof.

### BACKGROUND OF THE INVENTION

Platycladus orientalis leaves are dried branches and leaves of Platycladus orientalis, which have the effect of cooling blood and stopping bleeding. They are often used clinically to stop bleeding, relieve cough, eliminate phlegm and fight tuberculosis. Further, Platycladus orientalis leaves also have certain hair-growing benefits. Due to the numerous benefits of Platycladus orientalis leaves, how to utilize them effectively has become a major research topic.

Platycladus orientalis leaves are extracted in prior art for subsequent development and utilization. However, the Platycladus orientalis extracts obtained in prior art have poor system stability and low flavonoid content. Furthermore, the flavonoids in the system are prone to discoloration, which can lead to poor quality in finished products when used in subsequent applications, thus the application of Platycladus orientalis extracts is limited.

### SUMMARY OF THE INVENTION

One object of the present disclosure is to provide a Platycladus orientalis extract having good system stability and high flavonoid content.

Another object of the present disclosure is to provide a method of preparing a Platycladus orientalis extract.

Yet another object of the present disclosure is to provide use of a Platycladus orientalis extract in preparing a product for treating and/or preventing alopecia.

In order to achieve the above-mentioned objects of the present disclosure, in a first aspect, the present disclosure provides a Platycladus orientalis extract, comprising the following components in parts by mass: 0.121-0.902 parts of isoquercetin, 1.113-2.420 parts of afzelin, 2.825-4.481 parts of amentoflavone, 0.090-2.234 parts of pinusolide, and 510.61-759.44 parts of D-(-)-Quinic acid.

In a specific embodiment of the present disclosure, in the Platycladus orientalis extract, the percentages of isoquercetin, afzelin and amentoflavone in the total flavonoids are 0.30% to 1.00%, 0.20% to 1.90% and 3.50% to 3.90%, respectively.

In a specific embodiment of the present disclosure, the Platycladus orientalis extract further comprises at least one of propanediol and butanediol; and the Platycladus orientalis extract may comprise PEG-40 hydrogenated castor oil and/or disodium EDTA. Furthermore, in the Platycladus orientalis extract, the mass fraction of the PEG-40 hydrogenated castor oil is 0.2% to 0.6%, and the mass fraction of the disodium EDTA is 0.1% to 0.2%.

The present disclosure provides a method for preparing a Platycladus orientalis extract, comprising:

(a) extracting Platycladus orientalis leaves with an aqueous ethanol solution added as an extraction solvent in a material-to-liquid ratio of 1:10 to 1:30 at 25 to 85°C for 1 to 3 hours; and
(b) adjusting the pH of the extracted liquid obtained by the extraction to 8.3 to 9.5, stirring and then fine-filtering the extracted liquid into a filtrate, and then adjusting the pH of the filtrate to 7.0 ± 0.5.

In a specific embodiment of the present disclosure, the volume fraction of ethanol in the aqueous ethanol solution is 50% to 80%, preferably 50% to 60%.

In a specific embodiment of the present disclosure, the material-to-liquid ratio (m/V) is 1:10 to 1:20, preferably 1:15.

In a specific embodiment of the present disclosure, the extraction temperature is 45 to 85°C, preferably 65 to 85°C, and the extraction duration is 1 to 2.5 hours.

In a specific embodiment of the present disclosure, the Platycladus orientalis leaves are extracted in a coarse powder form, which has a particle size of 50-mesh sieve retained.

In a specific embodiment of the present disclosure, prior to the extraction, the Platycladus orientalis leaves are soaked in the extraction solvent; and the soaking duration is 0.5 to 1 hour.

In a specific embodiment of the present disclosure, the extraction is repeated 1 to 2 times.

In a specific embodiment of the present disclosure, the method further comprises concentrating the filtrate with pH adjusted to 7.0 ± 0.5 and performing reconstitution in an aqueous propanediol solution or an aqueous butanediol solution.

In a specific embodiment of the present disclosure, the amount of the aqueous propanediol solution or the aqueous butanediol solution used is 4 to 12 times the weight of the crude drug.

In a specific embodiment of the present disclosure, the mass fraction of propanediol in the aqueous propanediol solution is 20% to 40%, and the mass fraction of butanediol in the aqueous butanediol solution is 20% to 60%.

In a specific embodiment of the present disclosure, the method further comprises adding PEG-40 hydrogenated castor oil and/or disodium EDTA to the reconstituted liquid to obtain a liquid preparation. Furthermore, in the liquid preparation, the mass fraction of PEG-40 hydrogenated castor oil is 0.2% to 0.6%, and the mass fraction of disodium EDTA is 0.1% to 0.2%.

In a specific embodiment of the present disclosure, the reconstitution comprises stirring the material with the reconstitution solvents for 30-40 minutes, followed by fine-filtration.

In another specific embodiment of the present disclosure, the method further comprises concentrating the filtrate with pH adjusted to 7.0 ± 0.5 until the solids content reaches 6%-12%, and then performing spray-drying to obtain a powder. Furthermore, the inlet temperature during the spray-drying is 140-155°C.

The present disclosure also provides a method for improving the stability of a Platycladus orientalis extract, comprising:

adjusting the pH of the Platycladus orientalis extract to 8.3-9.5, stirring and then fine-filtering the extract into a filtrate, and then adjusting the pH of the filtrate to 7.0 ± 0.5.

In a specific embodiment of the present disclosure, the method further comprises concentrating the filtrate with pH adjusted to 7.0 ± 0.5 and performing reconstitution of the resultant in an aqueous propanediol solution or an aqueous butanediol solution.

The present disclosure also provides use of any one of the aforementioned Platycladus orientalis extracts or the Platycladus orientalis extract prepared according to any one of the aforementioned methods for preparing the Platycladus orientalis extract in the preparation of a product for treating and/or preventing alopecia.

Compared with the prior art, the present disclosure has the following beneficial effects:

(1) The present disclosure provides a Platycladus orientalis extract comprising a certain amount of isoquercetin, afzelin, amentoflavone, pinusolide and D-(-)-Quinic acid, which has the efficacy of preventing and treating alopecia and can be used as an alopecia-preventing raw material in alopecia-preventing products;

(2) The present disclosure discloses a method for preparing the Platycladus orientalis extract, which reduces the impurity content while increasing the content of target active ingredients and retaining D-(-)-Quinic acid by subjecting the mother liquid after extraction to a certain impurity removal treatment, thereby achieving selective removal of impurity; and

(3) In the method of preparing the Platycladus orientalis extract disclosed in the present disclosure, the content of the active ingredient flavonoids is increased by impurity removal together with reconstitution, and the stability of the system is improved, ensuring that the extract does not tend to change color.

## BRIEF DESCRIPTION OF THE DRAWINGS

In order to more clearly illustrate the specific embodiments of the present disclosure or the technical solutions in the prior art, the following briefly introduces drawings required for the specific embodiments or the description of the prior art. Apparently, the drawings described below are some embodiments of the present disclosure. For an ordinary skilled in the art, other drawings can be obtained based on these drawings without any creative efforts.

Figure 1 shows the dose-effect curve of finasteride's inhibitory effect on 5α-reductase;
Figure 2 shows the dose-effect curve of inhibitory effect of the sample of Example 52 on 5α-reductase; and
Figure 3 shows the results of the Malassezia antibacterial test according to the disclosure.

## DETAILED DESCRIPTION OF THE INVENTION

The Platycladus orientalis extracts in the prior art have the following disadvantages:

(1) they have poor system stability and low flavonoid content, and the flavonoids in the system are prone to discoloration, which can lead to poor quality in finished products when used in subsequent applications; and
(2) when removing impurities or enriching effective substances, one or more effective components will be concomitantly removed, and a selective removal of impurity is not possible.

The present disclosure provides a Platycladus orientalis extract, comprising the following components in parts by mass:
0.121-0.902 parts of isoquercetin, 1.113-2.420 parts of afzelin, 2.825-4.481 parts of amentoflavone, 0.090-2.234 parts of pinusolide, and 510.61-759.44 parts of D-(-)-Quinic acid.

For example, in various embodiments, the amount of each component in the Platycladus orientalis extract can be exemplified as follows:

the amount of isoquercetin can be 0.121 parts, 0.15 parts, 0.2 parts, 0.3 parts, 0.4 parts, 0.5 parts, 0.6 parts, 0.7 parts, 0.8 parts, 0.9 parts, 0.902 parts, or in a range consisting of any two thereof;
the amount of afzelin can be 1.113 parts, 1.2 parts, 1.5 parts, 1.8 parts, 2 parts, 2.2 parts, 2.420 parts, or in a range consisting of any two thereof;
the amount of amentoflavone can be 2.825 parts, 3 parts, 3.2 parts, 3.5 parts, 3.8 parts, 4 parts, 4.2 parts, 4.481 parts, or in a range consisting of any two thereof;
the amount of pinusolide can be 0.090 parts, 0.1 parts, 0.5 parts, 1 part, 1.5 parts, 2 parts, 2.234 parts, or in a range consisting of any two thereof; and
the amount of D-(-)-Quinic acid can be 510.61 parts, 520 parts, 550 parts, 580 parts, 600 parts, 650 parts, 700 parts, 750 parts, 759.44 parts, or in a range consisting of any two thereof.

In a specific embodiment of the present disclosure, in the Platycladus orientalis extract, the percentages of isoquercetin, afzelin and amentoflavone in the total flavonoids are 0.30% to 1.00%, 0.20% to 1.90% and 3.50% to 3.90%, respectively.

For example, in various embodiments, in the Platycladus orientalis extract, the percentage of isoquercetin in the total flavonoids can be 0.3%, 0.4%, 0.5%, 0.6%, 0.7%, 0.8%, 0.9%, 1% or in a range consisting of any two thereof; the percentage of afzelin in the total flavonoids can be 0.2%, 0.5%, 0.8%, 1%, 1.2%, 1.5%, 1.8%, 1.95% or in a range consisting of any two thereof; and the percentage of amentoflavone in the total flavonoids can be 3.5%, 3.6%, 3.7%, 3.8%, 3.9% or in a range consisting of any two thereof.

The total flavonoids refer to all flavonoid components in the Platycladus orientalis extract.

In a specific embodiment of the present disclosure, the Platycladus orientalis extract further comprises at least one of propanediol, butanediol, PEG-40 hydrogenated castor oil and disodium EDTA. Furthermore, in the Platycladus orientalis extract, the mass fraction of the PEG-40 hydrogenated castor oil is 0.2% to 0.6%, and the mass fraction of the disodium EDTA is 0.1% to 0.2%.

For example, in various embodiments, in the Platycladus orientalis extract, the mass fraction of the PEG-40 hydrogenated castor oil can be 0.2%, 0.3%, 0.4%, 0.5%, 0.6% or in a range consisting of any two thereof, and the mass fraction of the disodium EDTA can be 0.1%, 0.12%, 0.15%, 0.18%, 0.2% or in a range consisting of any two thereof.

The present disclosure provides a method for preparing a Platycladus orientalis extract, comprising:

(a) extracting Platycladus orientalis leaves, with an aqueous ethanol solution added as an extraction solvent, in a material-to-liquid ratio of 1:10 to 1:30 at 25 to 85°C for 1 to 3 hours; and
(b) adjusting the pH of the extracted liquid obtained by the extraction to 8.3 to 9.5, stirring and then fine-filtering the extracted liquid into a filtrate, and then adjusting the pH of the filtrate to 7.0 $\pm$ 0.5.

In practice, reagents used to adjust the pH may include sodium hydroxide and/or citric acid. Sodium hydroxide and citric acid may be added in the form of aqueous solution. The mass fraction of sodium hydroxide or citric acid in the aqueous solution can be adjusted according to actual needs, such as, but not limited to, 10%.

The present disclosure utilizes the above-mentioned extraction conditions to achieve increased flavonoid yield with less raw material and time cost.

For example, in various embodiments, the parameters of the preparation method may be exemplified as follows.

the material-to-liquid ratio can be 1:10, 1:12, 1:15, 1:18, 1:20, 1:22, 1:25, 1:28, 1:30, or in a range consisting of any two thereof;
the extraction temperature can be 25°C, 30°C, 35°C, 40°C, 45°C, 50°C, 55°C, 60°C, 65°C, 70°C, 75°C, 80°C, or in a range consisting of any two thereof;
the extraction duration can be 1 hour, 1.5 hours, 2 hours, 2.5 hours, or 3 hours. or in a range consisting of any two thereof;

in step (b), the pH of the extracted liquid is first adjusted to 8.3, 8.4, 8.5, 8.6, 8.7, 8.8, 8.9, 9, 9.1, 9.2, 9.3, 9.4, 9.5, 9.5, or in a range consisting of any two thereof; after the fine-filtration, the pH is adjusted to 6.5, 6.6, 6.7, 6.8, 6.9, 7.0, 7.1, 7.2, 7.3, 7.4, 7.5, or in a range consisting of any two thereof.

The extracted liquid obtained from Platycladus orientalis leaves contains a large amount of tannins, which can affect the stability of the system. The impurity removal process disclosed herein effectively removes tannins, significantly reducing the turbidity of the system and facilitating filtration after impurity removal. Furthermore, the impurity removal process retains a certain tannic acid substance, D-(-)-Quinic acid, in the system, achieving selective removal of impurity and thereby increasing the content of active ingredients in the extract liquid.

In a specific embodiment of the present disclosure, the volume fraction of ethanol in the aqueous ethanol solution is 50% to 80%, preferably 50% to 60%.

For example, in various embodiments, the volume fraction of ethanol in the aqueous ethanol solution can be 50%, 55%, 60%, 65%, 70%, 75%, 80%, or in a range consisting of any two thereof.

In a specific embodiment of the present disclosure, the material-to-liquid ratio (m/V) is 1:10 to 1:20, preferably 1:15.

In a specific embodiment of the present disclosure, the extraction temperature is 45 to 85°C, preferably 65 to 85°C, and the extraction duration is 1 to 2.5 hours.

The present disclosure utilizes the above-mentioned extraction parameters to further increase the flavonoid yield in the Platycladus orientalis extract.

In a specific embodiment of the present disclosure, in step (b), the stirring time is 0.5 to 1 hour.

In a specific embodiment of the present disclosure, the Platycladus orientalis leaves are extracted in a coarse powder form, which has a particle size of 50-mesh sieve retained.

The present disclosure has found in study that if the particle size of the raw material used for extraction is too small, filtration becomes difficult, resulting in significant loss of active ingredients and an excessively dark color in the finished product. It can be seen from the study that using coarse powder with a particle size of 50-mesh sieve retained can ensure effective extraction of the active ingredient while minimizing active ingredient loss during filtration.

In a specific embodiment of the present disclosure, prior to extraction, the Platycladus orientalis leaves are soaked in the extraction solvent; and the soaking duration is 0.5 to 1 hour.

In a specific embodiment of the present disclosure, the extraction is repeated 1-2 times.

In a specific embodiment of the present disclosure, the method further comprises concentrating the filtrate with pH adjusted to 7.0 ± 0.5 and performing reconstitution in an aqueous propanediol solution or an aqueous butanediol solution.

In practice, the concentration can be performed using conventional reduced-pressure concentration to remove ethanol therein. Using an aqueous propanediol solution or an aqueous butanediol solution as the reconstitution solvent can significantly reduce the turbidity of the reconstituted system, increase the flavonoid content in the reconstituted system, and ensure the stability of the system.

In a specific embodiment of the present disclosure, the amount of the aqueous propanediol solution or the aqueous butanediol solution used is 4 to 12 times the weight of the crude drug.

For example, in various embodiments, based on the amount of crude drug, the amount of the aqueous propanediol solution or the aqueous butanediol solution can be 4 times, 5 times, 6 times, 7 times, 8 times, 9 times, 10 times, 11 times, or 12 times the weight of the crude drugs, or a range of times consisting of any two thereof.

In a specific embodiment of the present disclosure, the mass fraction of propanediol in the aqueous propanediol solution is 20% to 40%, and the mass fraction of butanediol in the aqueous butanediol solution is 20% to 60%.

For example, in various embodiments, the mass fraction of propanediol in the aqueous propanediol solution can be 20%, 25%, 30%, 35%, 40%, or any two thereof; and the mass fraction of butanediol in the aqueous butanediol solution can be 20%, 25%, 30%, 35%, 40%, 45%, 50%, 55%, 60%, or any two thereof.

In practice, the butanediol can include any one or more of 1,2-butanediol, 1,3-butanediol, and 1,4-butanediol; and the propanediol can include 1,2-propanediol and/or 1,3-propanediol.

In a specific embodiment of the present disclosure, the method further comprises adding PEG-40 hydrogenated castor oil and/or disodium EDTA to the reconstituted liquid to obtain a liquid preparation. Furthermore, in the liquid preparation, the mass fraction of PEG-40 hydrogenated castor oil is 0.2% to 0.6%, and the mass fraction of disodium EDTA is 0.1% to 0.2%.

For example, in various embodiments, in the liquid preparation, the mass fraction of the PEG-40 hydrogenated castor oil can be 0.2%, 0.25%, 0.3%, 0.35%, 0.4%, 0.45%, 0.5%, 0.55%, 0.6% or in a range consisting of any two thereof, and in the liquid preparation, the mass fraction of the disodium EDTA can be 0.1%, 0.12%, 0.14%, 0.15%, 0.16%, 0.18%, 0.2% or in a range consisting of any two thereof.

In a specific embodiment of the present disclosure, the reconstitution comprises stirring with the aqueous propanediol solution or the aqueous butanediol solution for 30-40 minutes, followed by fine-filtration.

In practice, the material after fine-filtration is sterilized at 85-88°C for 40 minutes.

In another specific embodiment of the present disclosure, the method further comprises concentrating the filtrate with pH adjusted to 7.0 ± 0.5 until the solids content reaches 6%-12%, and then performing spray-drying to obtain a powder. Furthermore, the inlet temperature during the spray-drying is 140-155°C.

For example, in various embodiments, the solids content can reach 6%, 7%, 8%, 9%, 10%, 11%, 12%, or in a range consisting of any two thereof. During the spray-drying, the inlet temperature may be 140°C, 145°C, 150°C, 155°C, or in a range consisting of any two thereof.

The present disclosure also provides a method for improving the stability of a Platycladus orientalis extract, comprising:

adjusting the pH of the Platycladus orientalis extract to 8.3-9.5, stirring and then fine-filtering the extract into a filtrate, and then adjusting the pH of the filtrate to 7.0 ± 0.5.

In a specific embodiment of the present disclosure, the method further comprises concentrating the filtrate with pH adjusted to 7.0 ± 0.5 and performing reconstitution in an aqueous propanediol solution or an aqueous butanediol solution.

The above method can significantly reduce the impurity content in the Platycladus orientalis extract system while ensuring the effective retention of active ingredients such as D-(-)-Quinic acid.

The present disclosure also provides use of any one of the aforementioned Platycladus orientalis extracts or the Platycladus orientalis extract prepared according to any one of the aforementioned methods for preparing the Platycladus orientalis extract in the preparation of a product for treating and/or preventing alopecia.

In a specific embodiment of the present disclosure, the Platycladus orientalis extract is used in the preparation of a product for treating alopecia caused by Malassezia.

The following is a list of some of the raw materials used in the following specific examples, but is not limited to the following:

Platycladus orientalis leaves, origin: Henan (province); supplier: Beijing Qiancao Chinese Medicine Pieces Co., Ltd.; 1,3-propanediol, origin: United States; supplier: Kosfa International Trading (Shanghai) Co., Ltd.; PEG-40 hydrogenated castor oil, origin: Germany; supplier: BASF; and

Disodium EDTA, origin: Nantong, China; supplier: Nantong Aokai Biotechnology Development Co., Ltd.

Total flavonoids assay: The total flavonoid yield in Platycladus orientalis extract was determined using the $NaNO_2$-$Al(NO_3)_3$ colorimetric method.

The flavonoid yield in the following examples was calculated as follows: flavonoid content × total extract (or extract liquid) mass/ raw material mass.

Comprehensive ingredient analysis: Identification was performed using HPLC-MS/MS (high performance liquid chromatography-mass spectrometry).

**Examples 1-5**

The examples provide method for preparing a Platycladus orientalis extract, comprising:

(1) removing foreign matter from Platycladus orientalis leaves without crushing, extracting the treated Platycladus orientalis leaves with a 40-80%v/v aqueous ethanol solution added as an extraction solvent in a material-to-liquid ratio of 1:15 (m/V) at 65°C for 2 hours, and coarse-filtering the extracted material to obtain an extracted liquid; and

(2) adjusting the pH of the extracted liquid obtained in step (1) to 9.3 to 9.5, stirring it for 0.5 hours and removing impurities, and then fine-filtering the extracted liquid to obtain a filtrate; adjusting the pH of the filtrate to 7.0±0.5.

The specific extraction solvents used in the preparation methods of Examples 1 to 5 and the flavonoid yields in the obtained filtrates are shown in Table 1.

Table 1 Comparison of different extraction solvents

| No. | Extraction solvent | Flavonoid content (mg/mL) | Extraction yield (%) |
|---|---|---|---|
| Example 1 | 40% aqueous ethanol solution | 1.28 | 1.80 |
| Example 2 | 50% aqueous ethanol solution | 1.92 | 2.77 |
| Example 3 | 60% aqueous ethanol solution | 1.85 | 2.70 |
| Example 4 | 70% aqueous ethanol solution | 1.61 | 2.37 |

(continued)

| No. | Extraction solvent | Flavonoid content (mg/mL) | Extraction yield (%) |
|---|---|---|---|
| Example 5 | 80% aqueous ethanol solution | 1.46 | 2.12 |

**Examples 6-10**

The examples provide methods for preparing a Platycladus orientalis extract, comprising:

(1) removing foreign matter from Platycladus orientalis leaves without crushing, extracting the treated Platycladus orientalis leaves with a 50%v/v aqueous ethanol solution added as an extraction solvent in a material-to-liquid ratio of 1:15 (m/V) at 65°C for 0.5-3 hours, and coarse-filtering the extracted material to obtain an extracted liquid; and
(2) adjusting the pH of the extracted liquid obtained in step (1) to 9.3 to 9.5, stirring it for 0.5 hours and removing impurities, and then fine-filtering the extracted liquid to obtain a filtrate; adjusting the pH of the filtrate to 7.0±0.5.

The specific extraction duration used in the preparation methods of Examples 6 to 10 and the flavonoid yields in the obtained filtrates are shown in Table 2.

Table 2 Comparison of different extraction duration

| No. | Extraction duration | Flavonoid content (mg/mL) | Extraction yield (%) |
|---|---|---|---|
| Example 6 | 0.5h | 1.22 | 1.98 |
| Example 7 | 1h | 1.87 | 2.67 |
| Example 8 | 1.5h | 1.85 | 2.70 |
| Example 2 | 2h | 1.92 | 2.77 |
| Example 9 | 2.5h | 1.87 | 2.68 |
| Example 10 | 3h | 1.80 | 2.63 |

**Examples 11-14**

The examples provide methods for preparing a Platycladus orientalis extract, comprising:

(1) removing foreign matter from Platycladus orientalis leaves without crushing, extracting the treated Platycladus orientalis leaves with a 50%v/v aqueous ethanol solution added as an extraction solvent in a material-to-liquid ratio of 1:10 to 1:30 (m/V) at 65°C for 2 hours, and coarse-filtering the extracted material to obtain an extracted liquid; and
(2) adjusting the pH of the extracted liquid obtained in step (1) to 9.3 to 9.5, stirring it for 0.5 hours and removing impurities, and then fine-filtering the extracted liquid to obtain a filtrate; adjusting the pH of the filtrate to 7.0±0.5.

The specific material-to-liquid ratios used in the preparation methods of Examples 11 to 14 and the flavonoid yields in the obtained filtrates are shown in Table 3.

Table 3 Comparison of different material-to-liquid ratios

| No. | Material-to-liquid ratio (m/V) | Flavonoid content (mg/mL) | Extraction yield (%) |
|---|---|---|---|
| Example 11 | 1:10 | 2.42 | 2.42 |
| Example 2 | 1:15 | 1.92 | 2.77 |
| Example 12 | 1:20 | 1.37 | 2.87 |
| Example 13 | 1:25 | 1.12 | 2.91 |
| Example 14 | 1:30 | 0.96 | 2.97 |

**Examples 15-17**

The examples provide methods for preparing a Platycladus orientalis extract, comprising:

(1) removing foreign matter from Platycladus orientalis leaves without crushing, extracting the treated Platycladus orientalis leaves with a 50%v/v aqueous ethanol solution added as an extraction solvent in a material-to-liquid ratio of 1:15 (m/V) at 25-85°C for 2 hours, and coarse-filtering the extracted material to obtain an extracted liquid; and

(2) adjusting the pH of the extracted liquid obtained in step (1) to 9.3 to 9.5, stirring it for 0.5 hours and removing impurities, and then fine-filtering the extracted liquid to obtain a filtrate; adjusting the pH of the filtrate to 7.0±0.5.

The specific extraction temperatures used in the preparation methods of Examples 15 to 17 and the flavonoid yields in the obtained filtrates are shown in Table 4.

Table 4 Comparison of different extraction temperatures

| No. | Extraction temperature | Flavonoid content (mg/mL) | Extraction yield (%) |
| --- | --- | --- | --- |
| Example 15 | 25°C | 0.60 | 1.34 |
| Example 16 | 45°C | 1.64 | 2.06 |
| Example 2 | 65°C | 1.92 | 2.77 |
| Example 17 | 85°C | 2.00 | 2.91 |

**Examples 18-19**

The examples provide methods for preparing a Platycladus orientalis extract, comprising:

(1) removing foreign matter from Platycladus orientalis leaves without crushing, soaking the treated Platycladus orientalis leaves with a 50%v/v aqueous ethanol solution added as an extraction solvent in a material-to-liquid ratio of 1:15 (m/V) for 0-1 hour and then performing extraction at 65°C for 2 hours, and coarse-filtering the extracted material to obtain an extracted liquid; and

(2) adjusting the pH of the extracted liquid obtained in step (1) to 9.3 to 9.5, stirring it for 0.5 hours and removing impurities, and then fine-filtering the extracted liquid to obtain a filtrate; adjusting the pH of the filtrate to 7.0±0.5.

The specific soaking durations used in the preparation methods of Examples 18 to 19 and the flavonoid yields in the obtained filtrates are shown in Table 5.

Table 5 Comparison of different soaking durations

| No. | Soaking duration | Flavonoid content (mg/mL) | Extraction yield (%) |
| --- | --- | --- | --- |
| Example 2 | 0h | 1.92 | 2.77 |
| Example 18 | 0.5h | 1.92 | 2.80 |
| Example 19 | 1h | 1.93 | 2.82 |

**Examples 20-21**

The examples provide methods for preparing a Platycladus orientalis extract, comprising:

(1) removing foreign matter from Platycladus orientalis leaves without crushing, extracting the treated Platycladus orientalis leaves with a 50%v/v aqueous ethanol solution added as an extraction solvent in a material-to-liquid ratio of 1:15 (m/V) once and twice respectively at 85°C for 1 h each, and coarse-filtering the extracted material to obtain an extracted liquid; and

(2) adjusting the pH of the extracted liquid obtained in step (1) to 9.3 to 9.5, stirring it for 0.5 hours and removing impurities, and then fine-filtering the extracted liquid to obtain a filtrate for detection; adjusting the pH of the filtrate to 7.0±0.5.

The specific extraction times used in the preparation methods of Examples 20 to 21 and the flavonoid yields in the obtained filtrates are shown in Table 6.

# EP 4 691 473 A1

Table 6 Comparison of different extraction times

| No. | Extraction times | Flavonoid content (mg/mL) | Extraction yield (%) |
|---|---|---|---|
| Example 20 | 1 | 1.93 | 2.90 |
| Example 21 | 2 | 2.35 | 3.53 |

**Examples 22-26**

The examples provide methods for preparing a Platycladus orientalis extract, comprising:

(1) crushing Platycladus orientalis leaves with foreign matter removed; subjecting the resultant to sieving to obtain a sieve residue, such as a sieve residue retained on 10-mesh , between 10-mesh and 30-mesh (a sieve residue passed 10-mesh sieve and retained on 30-mesh sieve), between 30-mesh and 50-mesh (a sieve residue passed 30-mesh sieve and retained on 50-mesh sieve), between 50-mesh and 100-mesh (a sieve residue passed 50-mesh sieve and retained on 100-mesh sieve), and particles passed the sieve of 100-mesh; weighing each of the sieve residues (powders of Platycladus orientalis leaves) having different particle sizes respectively, extracting each of them with a 50%v/v aqueous ethanol solution added as an extraction solvent in a material-to-liquid ratio of 1:15 (m/V) at 65°C for 2 hours, and coarse-filtering the extracted material to obtain an extracted liquid; and among them, with respect to the raw material retained on the 10-mesh sieve, the maximum particle size thereof is about 1 cm (that is, the raw material particle size meets the requirements of being retained on a 10-mesh sieve and is ≤1 cm); and
(2) adjusting the pH of the extracted liquid obtained in step (1) to 9.3 to 9.5, stirring it for 0.5 hours and removing impurities, and then fine-filtering the extracted liquid to obtain a filtrate for detection; adjusting the pH of the filtrate to 7.0±0.5.

The specific particle size of Platycladus orientalis leaves used in the preparation methods of Examples 22 to 26 and the flavonoid yields in the obtained filtrates are shown in Table 7.

Table 7 Comparison of different particle sizes

| No. | Particle size | Appearance | Flavonoid content (mg/mL) | Extraction yield (%) |
|---|---|---|---|---|
| Example 22 | Retained on 10-mesh sieve | Slightly light color, stable | 1.75 | 2.80 |
| Example 23 | Between 10-mesh and 30-mesh | Slightly dark color, stable | 1.93 | 3.09 |
| Example 24 | Between 30-mesh and 50-mesh | Dark color, stable | 1.83 | 2.93 |
| Example 25 | Between 50-mesh and 100-mesh | Very dark color, precipitation appeared after 3 days | 1.69 | 2.70 |
| Example 26 | Passed 100-mesh sieve | Very dark color, a lot of precipitation appeared after 1 days | 1.33 | 2.13 |
| Example 2 | Without crushing | Light color, stable | 1.92 | 2.77 |

**Examples 27-30**

The examples provide methods for preparing a Platycladus orientalis extract, comprising:

(1) removing foreign matter from Platycladus orientalis leaves without crushing, extracting the treated Platycladus orientalis leaves with a 50%v/v aqueous ethanol solution added as an extraction solvent in a material-to-liquid ratio of 1:15 (m/V) at 85°C for 1 hour, and coarse-filtering the extracted material to obtain an extracted liquid;
(2) dividing the extracted liquid obtained in step (1) into 4 equal parts, in which the first part was adjusted to pH 8.3-8.5, the second part was adjusted to pH 9.3-9.5, third part was adjusted to pH 10.3-10.5, the three parts being stirred for 0.5 h and removing impurities and proceed to the next step, and the fourth part was not adjusted for pH and directly proceeded to the next step; and
(3) fine-filtering the treated extract obtained in step (2) to obtain a filtrate; adjusting the pH of the filtrate to 7.0±0.5.

9

The specific pH adjustment used in the preparation methods of Examples 27 to 30 and the flavonoid yields in the obtained filtrates are shown in Table 8.

Table 8 Comparison of different impurity removal conditions

| No. | pH adjustment | Condition | Flavonoid content (mg/mL) | Extraction yield (%) |
|---|---|---|---|---|
| Example 27 | 8.3-8.5 | No precipitation | 1.62 | 2.59 |
| Example 28 | 9.3-9.5 | No precipitation | 1.93 | 2.90 |
| Example 29 | 10.3-10.5 | No precipitation, severe color darkening | 1.60 | 2.56 |
| Example 30 | No pH adjustment | A lot of precipitation appeared after 1 days | 1.54 | 2.46 |

**Examples 31-37**

The examples provide methods for preparing a Platycladus orientalis extract, comprising:

(1) crushing Platycladus orientalis leaves, subjecting the resultant to a sieving to obtain a sieve residue retained on 10-mesh sieve and soaking the treated Platycladus orientalis leaves with a 50%v/v aqueous ethanol solution added as an extraction solvent in a material-to-liquid ratio of 1:15 (m/V) for 0.5 hours and then performing extraction at 85°C for 1 hour, and coarse-filtering the extracted material to obtain an extracted liquid; and
(2) adjusting the pH of the extracted liquid obtained in step (1) to 9.3 to 9.5, stirring it for 0.5 hours and removing impurities, and then fine-filtering the extracted liquid to obtain a filtrate with a turbidity ≤3; adjusting the pH of the filtrate to 7.0±0.1; and dividing the homogeneously mixed filtrate into 7 equal parts; and
(3) concentrating each of those parts obtained in step (2) until it reached the crude drug content ±5%, adding different reconstitution solvents in an amount 9 times the crude drug amount for reconstitution, mechanically stirring the resultants at room temperature for 30 minutes and filtering it to obtain a clear solution, sterilizing the solution at 85°C for 40 minutes, detecting the flavonoid content, and recording the dissolution of the final products and the transparency of the appearance.

The specific reconstitution solvents used in the preparation methods of Examples 31 to 37, as well as the properties of the final products, flavonoid contents and flavonoid yields are shown in Table 9.

Table 9 Comparison of different reconstitution solvents

| No. | Reconstitution solvent | Appearance | Flavonoid content (mg/mL) | Flavonoid yield (%) |
|---|---|---|---|---|
| Example 31 | 1,3-aqueous butanediol solution of 20% mass fraction | Transparent solution, good stability for 3 months | 0.87 | 0.87 |
| Example 32 | 1,3-aqueous propanediol solution of 20% mass fraction | Transparent solution, no change for 3 months | 1.09 | 1.09 |
| Example 33 | 1,3-aqueous butanediol solution of 40% mass fraction | Transparent solution, no change for 3 months | 0.91 | 0.91 |
| Example 34 | 1,3-aqueous propanediol solution of 40% mass fraction | Transparent solution, no change for 3 months | 1.03 | 1.03 |
| Example 35 | 1,3-aqueous butanediol solution of 60% mass fraction | Transparent solution, no change for 3 months | 0.99 | 0.99 |

(continued)

| No. | Reconstitution solvent | Appearance | Flavonoid content (mg/mL) | Flavonoid yield (%) |
|---|---|---|---|---|
| Example 36 | 1,3-aqueous propanediol solution of 60% mass fraction | Transparent solution, no change for 3 months | 1.02 | 1.02 |
| Example 37 | Pure water | Transparent solution, a lot of precipitation appeared after standing for 1 day | 0.42 | 0.42 |

From the above results, it can be seen that when the reconstitution solvent is a 1,3-aqueous butanediol solution with a mass fraction of 20% to 60% or a 1,3-aqueous propanediol solution with a mass fraction of 20% to 40%, both the stability of the system and the yield of flavonoids can be guaranteed.

**Examples 38-41**

The examples provide methods for preparing a Platycladus orientalis extract, comprising:

(1) crushing Platycladus orientalis leaves, subjecting the resultant to a sieving to obtain a sieve residue retained on 10-mesh sieve and soaking the treated Platycladus orientalis leaves with a 50%v/v aqueous ethanol solution added as an extraction solvent in a material-to-liquid ratio of 1:15 (m/V) for 0.5 hours and then performing extraction at 85°C for 1 hour, and coarse-filtering the extracted material to obtain an extracted liquid; and

(2) adjusting the pH of the extracted liquid obtained in step (1) to 9.3 to 9.5, stirring it for 0.5 hours and removing impurities, and then fine-filtering the extracted liquid to obtain a filtrate with a turbidity ≤3; adjusting the pH of the filtrate to 7.0±0.1; and dividing the homogeneously mixed filtrate into 4 equal parts; and

(3) concentrating each of those parts obtained in step (2) until it reached the crude drug content ±5%, adding a 1,3-aqueous butanediol solution with a mass fraction of 20% in an amount 2, 4, 8, or 12 times the crude drug amount for reconstitution, mechanically stirring the resultants at room temperature for 30 minutes and filtering it to obtain a clear solution, sterilizing the solution at 85°C for 40 minutes, detecting the flavonoid content, and recording the dissolution of the final products and the transparency of the appearance.

The specific reconstitution solvent amount used in the preparation methods of Examples 38 to 41, as well as the properties of the final products, flavonoid contents and flavonoid yields are shown in Table 10.

Table 10 Comparison of different reconstitution solvent amounts

| No. | Reconstitution solvent amount | Appearance | Flavonoid content (mg/mL) | Flavonoid yield (%) |
|---|---|---|---|---|
| Example 38 | 2 folds | Slightly turbid liquid | 2.58 | 0.77 |
| Example 39 | 4 folds | Transparent solution, good stability for 3 months | 2.10 | *1.05* |
| Example 40 | 8 folds | Transparent solution, no change for 3 months | 1.32 | 1.18 |
| Example 41 | 12 folds | Transparent solution, no change for 3 months | 0.54 | 0.70 |

**Examples 42-45**

The examples provide methods for preparing a Platycladus orientalis extract, comprising:

(1) crushing Platycladus orientalis leaves, subjecting the resultant to a sieving to obtain a sieve residue retained on 10-mesh sieve and soaking the treated Platycladus orientalis leaves with a 50%v/v aqueous ethanol solution added as an extraction solvent in a material-to-liquid ratio of 1:15 (m/V) for 0.5 hours and then performing extraction at 85°C for 1 hour, and coarse-filtering the extracted material to obtain an extracted liquid;

(2) adjusting the pH of the extracted liquid obtained in step (1) to 9.3 to 9.5, stirring it for 0.5 hours and removing impurities, and then fine-filtering the extracted liquid to obtain a filtrate with a turbidity ≤3; and adjusting the pH of the filtrate to 7.0±0.1; and

(3) concentrating the filtrate obtained in step (2) until it reached the crude drug content ±5%, adding a 1,3-aqueous propanediol solution with a mass fraction of 20% in an amount 9 times the crude drug amount for reconstitution, mechanically stirring the resultants at room temperature for 30 minutes, dividing the liquids into 4 parts into which 0.2%, 0.4%, 0.5%, 0.6% (mass fraction of PEG-40 hydrogenated castor oil in the product) of PEG-40 hydrogenated castor oil was added respectively, filtering them to obtain clear solutions, sterilizing the solutions at 85°C for 40 minutes, detecting the flavonoid content, and recording the dissolution of the final products and the transparency of the appearance.

The specific PEG-40 hydrogenated castor oil amounts used in the preparation methods of Examples 42 to 45, as well as the properties of the final products, flavonoid contents and flavonoid yields are shown in Table 11.

Table 11 Comparison of different PEG-40 hydrogenated castor oil amounts

| No. | PEG-40 hydrogenated castor oil amount | Appearance | Flavonoid content (mg/mL) | Flavonoid yield (%) |
|---|---|---|---|---|
| Example 42 | 0.2% | Slightly light color, transparent solution, stable for 3 months | 1.08 | 1.08 |
| Example 43 | 0.4% | Slightly dark color, transparent solution, stable for 3 months | 1.11 | 1.11 |
| Example 44 | 0.5% | Slightly dark color, transparent solution, stable for 3 months | 1.41 | 1.41 |
| Example 45 | 0.6% | Dark color, transparent solution, stable for 3 months | 1.33 | 1.33 |

**Examples 46-47**

The examples provide methods for preparing a Platycladus orientalis extract, comprising:

(1) crushing Platycladus orientalis leaves, subjecting the resultant to a sieving to obtain a sieve residue retained on 10-mesh sieve and soaking the treated Platycladus orientalis leaves with a 50%v/v aqueous ethanol solution added as an extraction solvent in a material-to-liquid ratio of 1:15 (m/V) for 0.5 hours and then performing extraction at 85°C for 1 hour, and coarse-filtering the extracted material to obtain an extracted liquid;

(2) adjusting the pH of the extracted liquid obtained in step (1) to 9.3 to 9.5, stirring it for 0.5 hours and removing impurities, and then fine-filtering the extracted liquid to obtain a filtrate with a turbidity ≤3; and adjusting the pH of the filtrate to 7.0±0.1; and

(3) concentrating the filtrate obtained in step (2) until it reached the crude drug content ±5%, adding a 1,3-aqueous propanediol solution with a mass fraction of 20% in an amount 9 times the crude drug amount for reconstitution, mechanically stirring the resultants at room temperature for 30 minutes, adding 0.5% (mass fraction of PEG-40 hydrogenated castor oil in the liquid being 0.5%) of PEG-40 hydrogenated castor oil to the liquid for solubilization, then dividing the liquid into 2 parts into which 0.1% and 0.15% (mass fraction of Disodium EDTA) of Disodium EDTA for color protection was added respectively, filtering them to obtain clear solutions, sterilizing the solutions at 85°C for 40 minutes, detecting the flavonoid content, and recording the dissolution of the final products and the transparency of the appearance.

The specific Disodium EDTA amounts used in the preparation methods of Examples 46 to 47, as well as the properties of the final products, flavonoid contents and flavonoid yields are shown in Table 12.

Table 12 Comparison of different Disodium EDTA amounts

| No. | Disodium EDTA amount | Appearance | Flavonoid content (mg/mL) | Flavonoid yield (%) |
|---|---|---|---|---|
| Example 46 | 0.1% | Transparent solution with its color stable for 3 months | 1.39 | 1.39 |
| Example 47 | 0.2% | Transparent solution with its color stable for 3 months | 1.40 | 1.40 |

**Examples 48-51**

The examples provide methods for preparing a Platycladus orientalis extract, comprising:

(1) crushing Platycladus orientalis leaves, subjecting the resultant to a sieving to obtain a sieve residue retained on 10-mesh sieve and soaking the treated Platycladus orientalis leaves with a 50%v/v aqueous ethanol solution added as an extraction solvent in a material-to-liquid ratio of 1:15 (m/V) for 0.5 hours and then performing extraction at 85°C for 1 hour, and coarse-filtering the extracted material to obtain an extracted liquid;
(2) adjusting the pH of the extracted liquid obtained in step (1) to 9.3 to 9.5, stirring it for 0.5 hours and removing impurities, and then fine-filtering the extracted liquid to obtain a filtrate with a turbidity ≤3; and adjusting the pH of the filtrate to 7.0±0.1;
(3) concentrating the filtrate obtained in step (2) under negative pressure at 60±5°C to remove ethanol until the solids content of the concentrate reached 6% to 8%; and
(4) placing the concentrate in a spray-dryer with the inlet temperature controlled at 140°C, 145°C, 150°C, and 155°C, respectively, collecting the powder after drying for about 1 hour, and determining the flavonoid content therein.

The specific inlet temperatures used in the preparation methods of Examples 48 to 51, as well as the properties of the final products, flavonoid contents and flavonoid yields are shown in Table 13.

Table 13 Comparison of different inlet temperatures

| No. | Inlet temperature | Appearance | Flavonoid content (mg/g) | Flavonoid yield (%) |
|---|---|---|---|---|
| Example 48 | 140°C | Brown yellow powder | 94 | 0.97 |
| Example 49 | 145°C | Brown yellow powder | 98 | 1.04 |
| Example 50 | 150°C | Brown yellow powder | 100 | 1.08 |
| Example 51 | 155°C | Brown powder | 90 | 0.95 |

**Examples 52-53**

The examples provide methods for preparing a Platycladus orientalis extract, comprising:

(1) crushing Platycladus orientalis leaves, subjecting the resultant to a sieving to obtain a sieve residue retained on 10-mesh sieve and soaking the treated Platycladus orientalis leaves with a 50%v/v aqueous ethanol solution added as an extraction solvent in a material-to-liquid ratio of 1:15 (m/V) for 0.5 hours and then performing extraction at 85°C for 1 hour, and coarse-filtering the extracted material to obtain an extracted liquid;
(2) adjusting the pH of the extracted liquid obtained in step (1) to 9.3 to 9.5, stirring it for 0.5 hours and removing impurities, and then fine-filtering the extracted liquid to obtain a filtrate with a turbidity ≤3; adjusting the pH of the filtrate to 7.0±0.1; and dividing the filtrate into 2 parts;
(3) concentrating the parts obtained in step (2) under negative pressure at 60±5°C to remove ethanol until the solids content of the concentrates reached 8% to 10% and 10% to 12%, respectively; and
(4) placing the concentrates in a spray-dryer with the inlet temperature controlled at 140°C, collecting the powder after drying for about 1 hour, and determining the flavonoid content therein.

The specific solids content used in the preparation methods of Examples 52 to 53, as well as the properties of the final products, flavonoid contents and flavonoid yields are shown in Table 14.

Table 14 Comparison of different solids content

| No. | Solids content | Appearance | Flavonoid content (mg/g) | Flavonoid yield (%) |
|---|---|---|---|---|
| Example 48 | 6%-8% | Brown yellow powder (Susceptible to moisture) | 94 | 0.97 |
| Example 52 | 8%-10% | Brown yellow powder | 102 | 1.10 |
| Example 53 | 10%-12% | Brown yellow powder (great loss due to excessive wall adhesion) | 110 | 0.89 |

**Comparative Example 1**

Comparative example 1 provides a method for preparing a Platycladus orientalis extract, comprising:

(1) crushing Platycladus orientalis leaves, subjecting the resultant to a sieving to obtain a sieve residue retained on 10-mesh sieve and soaking the treated Platycladus orientalis leaves with a 50%v/v aqueous ethanol solution added as an extraction solvent in a material-to-liquid ratio of 1:15 (m/V) for 0.5 hours and then performing extraction at 85°C for 1 hour, and coarse-filtering the extracted material to obtain an extracted liquid;
(2) fine-filtering the extracted liquid obtained in step (1) to produce a filtrate, concentrating the filtrate under negative pressure at 60±5°C to remove ethanol until the solids content of the concentrate reached 8% to 10%; and
(3) placing the concentrate in a spray-dryer with the inlet temperature controlled at 140°C, and collecting the powder after drying for about 1 hour.

**Comparative Example 2**

Comparative example 2 provides a method for preparing a Platycladus orientalis extract, comprising:

(1) crushing Platycladus orientalis leaves, subjecting the resultant to a sieving so as to yield a fine powder of between 30-mesh and 50-mesh (retained on a 50-mesh sieve and passed a 30-mesh sieve) and soaking the powder with a 50%v/v aqueous ethanol solution added as an extraction solvent in a material-to-liquid ratio of 1:15 (m/V) for 0.5 hours and then performing extraction at 85°C for 1 hour, and coarse-filtering the extracted material to obtain an extracted liquid;
(2) adjusting the pH of the extracted liquid obtained in step (1) to 9.3 to 9.5, stirring it for 0.5 hours and removing impurities, and then fine-filtering the extracted liquid to obtain a filtrate; adjusting the pH of the filtrate to 7.0±0.1 without further filtration;
(3) concentrating the filtrate obtained in step (2) under negative pressure at 60±5°C to remove ethanol until the solids content of the concentrate reached 8% to 10%; and
(4) placing the concentrate in a spray-dryer with the inlet temperature controlled at 140°C, and collecting the powder after drying for about 1 hour.

Performance test results of the products obtained in Example 52 and Comparative Examples 1-2 are shown in Table 15.

Table 15 Performance test results of different products

| No. | Flavonoid content (mg/g) | Extraction yield (%) |
|---|---|---|
| Example 52 | 102 | 1.10 |
| Comparative Example 1 | 84.3 | 0.89 |
| Comparative Example 2 | 79.4 | 0.64 |

**Experimental Example 1**

$5\alpha$-Reductase Inhibition Test

(1) Test drug: Sample of Example 52

(2) Positive control drug: Finasteride, a product of Merck Sharp & Dohme Limited; Appearance: Film-coated tablets, white after removal of the coating; Specification: 5 mg/tablet; Batch number: 0022050. Storage conditions: light-shielded, airtight, and stored at room temperature.

(3) Drug preparation: The sample of Example 52 was tested at 10 concentrations: 35 mg/mL (solute: the sample of Example 52, solvent: 20% aqueous ethanol solution) was adopted as the initial concentration, and nine 2.5-fold gradient dilutions were made by using 20% aqueous ethanol solution; and five 2-fold gradient dilutions of the positive control finasteride were made by using anhydrous ethanol.

(4) Reagents: Reduced coenzyme II (NADPH), a product of Sigma, Catalog number N7505, Batch number 041939; testosterone (T), a product of Merck, Catalog number T-037; Batch number FE03112002; Tris buffer: Shanghai Yuanye Biotechnology Co., Ltd., Batch number J17HR10516A.

(5) Test instrument: ZDN microplate reader, a product of BioTek.

Test method:

Mouse liver was obtained by dissection, and was added into a Tris buffer at a ratio of 1:5 for homogenization at 0°C. The homogenized liver was centrifuged at 10000g for 15 minutes, the supernatant was centrifuged at 55000g for another hour, and then the supernatant was placed at 4°C for later use. Reagents and samples were added according to the reaction system. Sample of Example 52 and finasteride sample at each dilution were added 4 times. After incubation at 37°C for 60 minutes, the decrease in absorbance $\Delta A$ at 340nm was measured.

$$\text{Inhibition rate}\,(\%) = \frac{\text{blank}\,\Delta A - \text{sample}\,\Delta A}{\text{blank}\,\Delta A} \times 100$$

The 5$\alpha$-reductase reaction system is shown in Table 16:

Table 16 Reagent amount for the reaction system ($\mu$L)

| Material name | Amount added $\mu$L | Final concentration $\mu$mol/L |
|---|---|---|
| 5$\alpha$-reductase solution | 10 | - |
| T | 5 | 25 |
| NADPH | 10 | 100 |
| Drugs to be tested | 5 | |
| PBS | 220 | |

Test Results:

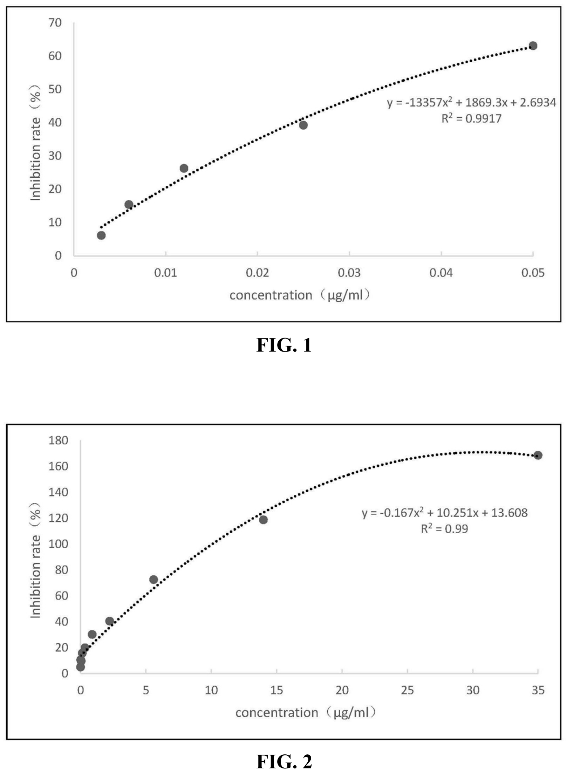

For 5$\alpha$-reductase, the inhibition rates and IC$_{50}$ values of finasteride at different concentration gradients and the samples of Example 52 at different concentration gradients are shown in Table 17, Figures 1, and 2.

Table 17 Inhibition rate and IC$_{50}$ of the samples of Example 52 and finasteride for 5$\alpha$-reductase

| Finasteride $\mu$g/mL | A | Inhibition rate | Sample of Example 52 mg/mL | A | Inhibition rate |
|---|---|---|---|---|---|
| 0.050 | 0.04 | 63.07% | 35.00 | -0.07 | 168.53% |
| 0.025 | 0.07 | 39.22% | 14.00 | -0.02 | 118.65% |
| 0.012 | 0.08 | 26.26% | 5.60 | 0.03 | 72.49% |
| 0.006 | $\Delta$ 0.09 | 15.40% | 2.24 | $\Delta$ 0.06 | 40.33% |
| 0.003 | 0.10 | 6.12% | 0.90 | 0.08 | 30.07% |
| | | | 0.36 | 0.09 | 19.81% |
| | | | 0.14 | 0.09 | 15.62% |
| | | | 0.06 | 0.10 | 9.56% |
| | | | 0.02 | 0.10 | 10.49% |
| | | | 0.01 | 0.10 | 4.90% |
| IC$_{50}$ | 0.033$\mu$g/mL | | IC$_{50}$ | 3.78mg/mL | |

From the above results, it can be seen that Example 52 sample has an inhibitory effect on $5\alpha$-reductase activity, with an $IC_{50}$ of 3.78 mg/mL. The $IC_{50}$ of the Platycladus orientalis extract in the reaction system is converted to 0.076 mg/mL, equivalent to a crude drug concentration of 0.72 mg/mL. In summary, the sample of Example 52 of the Platycladus orientalis extract of the present disclosure has a significant $5\alpha$-reductase inhibitory effect at a low concentration.

**Experimental Example** 2

Malassezia antibacterial test

Test materials:

(1) Experimental instruments

The main instruments used in this experiment are shown in Table 18.

Table 18 Main experimental instruments

| Instrument Name | Model | Manufacturer |
|---|---|---|
| microplate reader | infinite M200 PRO | Tecan |
| centrifuge | Centrifuge 5430 R | Eppendorf |
| Ultra-clean workbench | / | / |

(2) Experimental reagents

The main reagents used in this experiment are shown in Table 19.

Table 19 Main experimental reagents

| Reagent name | Manufacturer |
|---|---|
| Malt extract culture medium | Aoboxing |
| Sterile PBS | Gibco |

(3) Sample Information Table

The sample information for this experiment is shown in Table 20.

Table 20 Experimental sample information

| Sample name | Sample appearance | Sample concentration (mg/mL) |
|---|---|---|
| Comparative Example 1 Comparative Example 2 Example 52 | Light yellow powder | 50, 40, 30, 20, 10, and 5 |

Test Method:

Cultivation of Malassezia

A culture medium and Malassezia were mixed thoroughly by shaking until no sediment was present at the bottom. A sterile syringe was used to pipette 200-800 $\mu$L of the bacterial suspension into fresh culture medium and incubate at 30°C in a biochemical incubator. After 2-3 days of incubation, the sample was subcultured.

Microbroth Method for testing Antibacterial Activity

1. Preparation of Fresh Culture Medium

A sterile malt extract culture medium was transferred into a sterile 50mL centrifuge tube. The sterile malt extract culture medium was centrifuged at 25°C and 3500 rpm for 10 minutes. The supernatant was transferred to another sterile 50mL centrifuge tube for later use.

Each sample was diluted with fresh culture medium to a concentration of 50 mg/mL, 40 mg/mL, 30 mg/mL, 20 mg/mL, 10 mg/mL, or 5 mg/mL.

2. Extraction of Malassezia

The culture medium and Malassezia were mixed thoroughly by shaking and transferred to a sterile 50mL centrifuge tube. The mixture was centrifuged at 25°C and 1000 rpm for 30 seconds. The supernatant was transferred to a sterile 50 mL centrifuge tube and centrifuge at 25°C and 3500 rpm for 10 minutes.

3. Determination of the bacterial concentration

A microplate reader was set at a wavelength of 600 nm. 400 $\mu$L of the bacterial suspension was transferred into a 1.5 mL sterile centrifuge tube, to which 1 mL of PBS was added. 200 $\mu$L of the bacterial suspension was transferred from the 1.5 mL centrifuge tube containing 400 $\mu$L of the bacterial suspension into a standard 96-well plate. The OD600 value of the bacterial suspension was detected at 600 nm. If the OD600 value is > 1, dilution was performed with sterile PBS (starting with dilutions of 2x, 4x, 6x, and 8x). When the OD value reaches 1, proceed to the next step.

4. Dilution of the bacterial suspension

The required volume of bacterial suspension was calculated. The bacterial suspension (OD600 = 1) was diluted 160-fold with fresh culture medium.

5. Plating

In a sterile 96-well culture plate, 100$\mu$L of the diluted bacterial suspension was added to each test well. Then, 100$\mu$L of samples of varying concentrations were added to the wells. A control consisting of 100$\mu$L of sample of varying concentrations plus 100$\mu$L of malt extract medium was used. A blank control consisting of 200$\mu$L of malt extract medium without bacteria was used. A negative control consisting of 100$\mu$L of the diluted bacterial suspension plus 100$\mu$L of malt extract medium was used. A positive control consisting of 100$\mu$L of the diluted bacterial suspension plus 100$\mu$L of 4$\mu$g/mL ketoconazole was used. Each well was plated in triplicate.

The sterile 96-well culture plate was placed in a 30°C incubator. After 72 hours of incubation, the absorbance at 600nm was recorded.

6. Malassezia Inhibition Rate

$$\text{positive inhibition rate (\%)} = \left(1 - \frac{\text{positive control - blank control}}{\text{negative control - blank control}}\right) \times 100\%$$

$$\text{sample inhibition rate (\%)} = \left(1 - \frac{\text{sample - control}}{\text{negative control - blank control}}\right) \times 100\%$$

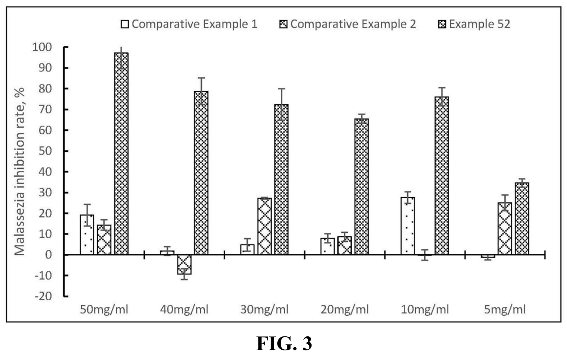

Test results: See Table 21 and Figure 3.

Table 21 Test results

| Sample name | 50mg/mL (5%) | 40mg/mL (4%) | 30mg/mL (3%) | 20mg/mL (2%) | 10mg/mL (1%) | 5mg/mL (0.5%) | Positive control (Ketoconazole 4μg/mL) |
|---|---|---|---|---|---|---|---|
| Comparative Example 1 | 19.09±5.26 | 1.89±2.08 | 4.79±3.06 | 7.97±2.19 | 27.58±2.78 | -1.2±1.27 | 95±3.59 |
| Comparative Example 2 | 14.37±2.56 | -9.26±2.57 | 27.24±0.39 | 8.69±2.18 | -0.14±2.49 | 25.04±3.72 | 95±3.59 |
| Example 52 | 97.21±7.6 | 78.75±6.42 | 72.38±7.55 | 65.44±2.18 | 76.03±4.37 | 34.60±1.99 | 95±3.59 |

From the above results, it can be seen that samples of Comparative Examples 1 and 2 had no Malassezia inhibitory effect at concentrations below 50 mg/mL, while the sample of Example 52 had a Malassezia inhibitory effect at concentrations above 10 mg/mL. In summary, the sample of Example 52 had a significant Malassezia inhibitory effect at low concentrations.

**Experimental Example 3**

Test for efficacy of Zebrafish embryo blood circulation promotion

Test method:

Healthy zebrafish embryos, 3 days post-fertilization, were selected.

1. Test grouping:

The test required a blank control (fish embryo culture medium/solvent solution), a positive control (caffeine solution), and a test article (the sample of Example 52).

2. Blank control setup:

40 fish embryos were randomly selected and transferred to a 6 cm culture dish. 8 mL of fish embryo culture medium was added.

3. Positive control setup:

40 fish embryos were randomly selected and transferred to a 6 cm culture dish. 8 mL of 0.05 g/L caffeine solution was added (the working solution was a 0.05 g/L caffeine solution prepared with fish embryo culture medium).

4. Test article treatment:

40 fish embryos were randomly selected and transferred to a 6 cm culture dish. 8 mL of test article solution (prepared by dissolving the test article directly in fish embryo culture medium) was added. The culture dish was incubated in a 28±1°C incubator for 2±0.5 hours.

5. Microscopic analysis of samples:

The fish embryos were anesthetized with a tricaine working solution (stock solution was prepared by dissolving 200mg of tricaine (analytical grade) in 48.95mL of water, refrigerated, and is valid for 30 days, and the working solution was prepared by diluting the stock solution to 0.04% with fish embryo culture medium before use) for 2 minutes. Video of the dorsal aorta of the fish embryos was taken under a microscope for 20 seconds.

6. Data and result calculation:

The relative blood flow velocity in the dorsal aorta of each fish embryo was determined using software such as DanioScope and averaged.

Calculation of results:

Calculation of the blood circulation promotion rate:

$$\text{Promotion rate} = (T - C) / C \times 100\%;$$

Where:

T represents average relative blood flow velocity of the dorsal aorta of fish embryos in the test article-treated group; and
C represents average relative blood flow velocity of the dorsal aorta of fish embryos in the blank control.

A two-tailed T-test was performed to compare the relative blood flow velocity of the dorsal aorta of fish embryos in the test article-treated group with the relative blood flow velocity of the dorsal aorta of the blank Control to obtain the p-value.
Test Results: See Table 22 for the test results.

Table 22 Test results

| Sample name | CB-2 | | |
|---|---|---|---|
| Sample ID | VI-20221844S-2 | | |
| Test | Test article concentratio n | Result | Evaluation of blood circulation promotion efficacy |
| Blood circulation promotion rate of the dorsal aorta of zebrafish embryos | 0.05% | 60% (p=0.0000014) | significant |
| | 0.01% | 22% (p=0.022) | significant |

From the above results, it can be seen that the Platycladus orientalis extract sample of the present disclosure can significantly promote the blood flow rate of zebrafish embryos and has a blood circulation promoting effect. In summary, the sample of Example 52 of the present disclosure has a significant blood circulation promoting effect at a low concentration, which to some extent proves that it has a nourishing and alopecia-preventing effect.

**Experimental Example 4**

A total constituent analysis of the samples of Examples 48-53 and Comparative Example 1 were performed. The contents of the main active ingredients are shown in Table 23.

Table 23 Total constituent analysis results of different products (powder mg/g)

| No. | Isoquercetin | afzelin | amentoflavone | pinusolide | D-(-)-Quinic acid |
|---|---|---|---|---|---|
| Example 48 | 0.675 | 1.426 | 2.997 | 0.090 | 510.61 |
| Example 49 | 0.702 | 1.643 | 2.630 | 1.763 | 623.87 |
| Example 50 | 0.823 | 1.834 | 3.712 | 2.009 | 686.76 |
| Example 51 | 0.121 | 1.113 | 2.852 | 1.023 | 521.33 |
| Example 52 | 0.850 | 1.925 | 3.981 | 1.834 | 560.61 |
| Example 53 | 0.902 | 2.420 | 4.481 | 2.234 | 759.44 |
| Comparative Example 1 | 0.321 | 1.203 | 3.320 | 0.120 | 709.44 |

The percentage of flavonoids in the total flavonoids in the Platycladus orientalis extract powder prepared in Example 52 and Comparative Example 1 was tested, and the results are shown in Table 24.

Table 24 Proportion of flavonoids in different products

| No. | Isoquercetin | afzelin | amentoflavone |
|---|---|---|---|
| Example 52 | 0.80% | 1.82% | 3.76% |
| Comparative Example 1 | 0.35% | 1.31% | 3.60% |

The above results indicate that the impurity removal process disclosed herein can increase the content of active ingredients and specifically retain the tannic acid, D-(-)-Quinic acid.

Industrial Applicability

The present disclosure provides a Platycladus orientalis extract, preparation method therefor and use thereof. Also provided is a Platycladus orientalis extract, comprising the following components in parts by mass: 0.121-0.902 parts of isoquercetin, 1.113-2.420 parts of afzelin, 2.825-4.481 parts of amentoflavone, 0.090-2.234 parts of pinusolide, and 510.61-759.44 parts of D-(-)-Quinic acid. The present disclosure provides a Platycladus orientalis extract comprising a certain amount of isoquercetin, afzelin, amentoflavone, pinusolide and D-(-)-Quinic acid, which has the efficacy of preventing and treating alopecia and can be used as an alopecia-preventing raw material in a product for preventing alopecia.

**Claims**

1. A Platycladus orientalis extract, comprising the following components in parts by mass: 0.121-0.902 parts of isoquercetin, 1.113-2.420 parts of afzelin, 2.825-4.481 parts of amentoflavone, 0.090-2.234 parts of pinusolide, and 510.61-759.44 parts of D-(-)-Quinic acid.

2. The Platycladus orientalis extract according to claim 1, wherein in the Platycladus orientalis extract, the percentages of isoquercetin, afzelin and amentoflavone in the total flavonoids are 0.30% to 1.00%, 0.20% to 1.90%, and 3.50% to 3.90%, respectively.

3. The Platycladus orientalis extract according to claim 1, wherein the Platycladus orientalis extract further comprises at least one of propanediol and butanediol.

4. The Platycladus orientalis extract according to claim 1, wherein the Platycladus orientalis extract further comprises PEG-40 hydrogenated castor oil and/or disodium EDTA.

5. The Platycladus orientalis extract according to claim 4, wherein in the Platycladus orientalis extract, the mass fraction of the PEG-40 hydrogenated castor oil is 0.2% to 0.6%, and the mass fraction of the disodium EDTA is 0.1% to 0.2%.

6. A method for preparing a Platycladus orientalis extract, comprising:

   (a) extracting Platycladus orientalis leaves with an aqueous ethanol solution added as an extraction solvent in a material-to-liquid ratio of 1:10 to 1:30 at 25 to 85°C for 1 to 3 hours; and
   (b) adjusting the pH of the extracted liquid obtained by the extraction to 8.3 to 9.5, stirring and then fine-filtering the extracted liquid into a filtrate, and then adjusting the pH of the filtrate to 7.0 ± 0.5.

7. The method according to claim 6, wherein the volume fraction of ethanol in the aqueous ethanol solution is 50% to 80%.

8. The method according to claim 6, comprising at least one of the following features:

   (1) the volume fraction of the ethanol is 50% to 60%;
   (2) the material-to-liquid ratio is 1:10 to 1:20;
   (3) the extraction temperature is 45 to 85°C;
   (4) the extraction duration is 1 to 2.5 hours;
   (5) the Platycladus orientalis leaves are extracted in a coarse powder form, which has a particle size of 50-mesh sieve retained;

(6) prior to the extraction, the Platycladus orientalis leaves are soaked in the extraction solvent and the soaking duration is 0.5 to 1 hour; and
(7) the extraction is repeated 1 to 2 times.

9. The method according to claim 6, further comprising concentrating the filtrate with pH adjusted to $7.0 \pm 0.5$ and performing reconstitution in an aqueous propanediol solution or an aqueous butanediol solution.

10. The method according to claim 9, wherein the amount of the aqueous propanediol solution or the aqueous butanediol solution used is 4 to 12 times the weight of the crude drug.

11. The method according to claim 9, wherein the mass fraction of propanediol in the aqueous propanediol solution is 20% to 40%, and the mass fraction of butanediol in the aqueous butanediol solution is 20% to 60%.

12. The method according to claim 9, further comprising adding PEG-40 hydrogenated castor oil and/or disodium EDTA to the reconstituted liquid to obtain a liquid preparation.

13. The method according to claim 12, wherein the mass fraction of the PEG-40 hydrogenated castor oil in the liquid preparation is 0.2% to 0.6%.

14. The method according to claim 12, wherein the mass fraction of disodium EDTA in the liquid preparation is 0.1% to 0.2%.

15. The method according to claim 6, further comprising concentrating the filtrate with pH adjusted to $7.0 \pm 0.5$ until the solids content reaches 6%-12%, and then performing spray-drying to obtain a powder.

16. The method according to claim 15, wherein the inlet temperature during the spray-drying is 140-155°C.

17. A method for improving the stability of a Platycladus orientalis extract, comprising adjusting the pH of the Platycladus orientalis extract to 8.3-9.5, stirring and then fine-filtering the extract into a filtrate, and then adjusting the pH of the filtrate to $7.0 \pm 0.5$.

18. The method according to claim 17, further comprising concentrating the filtrate with pH adjusted to 7.0:1: 0.5 and performing reconstitution in an aqueous propanediol solution or an aqueous butanediol solution.

19. Use of the Platycladus orientalis extract of any one of claims 1-5 or the Platycladus orientalis extract prepared according to the method of any one of claims 6-16 in the preparation of a product for treating and/or preventing alopecia.

**FIG. 1**

**FIG. 2**

**FIG. 3**

## INTERNATIONAL SEARCH REPORT

| | International application No. |
|---|---|
| | **PCT/CN2023/127216** |

| A. | CLASSIFICATION OF SUBJECT MATTER |
|---|---|

A61K31/7048(2006.01)i; A61K31/352(2006.01)i; A61K31/365(2006.01)i; A61K31/435(2006.01)i; A61K47/08(2006.01)i; A61K47/18(2017.01)i; A61K36/11(2006.01)i; A61P17/14(2006.01)i

According to International Patent Classification (IPC) or to both national classification and IPC

| B. | FIELDS SEARCHED |
|---|---|

Minimum documentation searched (classification system followed by classification symbols)

IPC:A61K，A61P

Documentation searched other than minimum documentation to the extent that such documents are included in the fields searched

Electronic data base consulted during the international search (name of data base and, where practicable, search terms used)

CNABS: CNTXT; ENTXTC; VEN; ENTXT; WOTXT; USTXT; CNKI; 万方, WANFANG: ISI Web of Science; Pubmed; CAPLUS: REGISTRY: 异槲皮苷, 异槲皮素, 异槲皮苷, 异槲皮, 异栎素, 阿福豆碱, 阿福豆苷, 穗花杉双黄酮, 阿曼托双黄酮, 红松内酯, 奎宁酸, 金鸡纳酸, 侧柏, 柏叶, 柏, 脱发, 生发, 乙醇, Isoquercetin, isotrifoliin, trifoliin, isoquercetrin, Afzelin, Afzeloside, Kaemperin, Kaempferol, amentoflavone, Pinusolide, Quininic, Concha, Platycladus, arborvitae, cacumen, platycladi, Biota orientali, Thuja orientalis, Thuja occidentalis, Chamaecyparis pisifera, anti-alopecia, hair-growth, hair loss, alcohol

| C. | DOCUMENTS CONSIDERED TO BE RELEVANT |
|---|---|

| Category* | Citation of document, with indication, where appropriate, of the relevant passages | Relevant to claim No. |
|---|---|---|
| A | CN 114028281 A (DONGGUAN BOTON FLAVOR & FRAGRANCE CO., LTD.) 11 February 2022 (2022-02-11) claims 1-10, and description, paragraph [0005] | 1-19 |
| A | CN 114948940 A (HAOXI (HAINAN) BIOLOGICAL TECHNOLOGY CO., LTD.) 30 August 2022 (2022-08-30) claims 1-8, and description, paragraph [0007] | 1-19 |
| A | KR 100739225 B1 (KWANGDUK SINYOUK) 13 July 2007 (2007-07-13) description, extract preparation example 2 | 1-19 |
| A | CN 110478296 A (HANGZHOU YAYAN COSMETICS CO., LTD.) 22 November 2019 (2019-11-22) claims 1-4 | 1-19 |
| A | CN 102885928 A (302 MILITARY HOSPITAL OF CHINA) 23 January 2013 (2013-01-23) claim 1 | 1-19 |

| ✓ Further documents are listed in the continuation of Box C. | ✓ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **24 January 2024** | **05 February 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/127216** |

### C. DOCUMENTS CONSIDERED TO BE RELEVANT

| Category* | Citation of document, with indication, where appropriate, of the relevant passages | Relevant to claim No. |
| --- | --- | --- |
| A | KR 20030052689 A (AMOREPACIFIC CORP.) 27 June 2003 (2003-06-27)<br>claims 1-6 | 1-19 |
| A | 中国人民解放军第四军医大学防治慢性气管炎药物药理研究组 (Non-official translation: Research Group of the Fourth Military Medical University of the Chinese People's Liberation Army on Pharmacology of Drugs for Prevention and Treatment of Chronic Tracheitis). "侧柏叶有效成份提取及药理实验结果 (Non-official translation: Extraction of Effective Components from Platycladus Orientalis Leaves and Results of Pharmacological Experiments)"<br>*Chongqing New Medicine*, 31 December 1972 (1972-12-31), pages 10-13<br>    page 10 | 1-19 |
| A | YE, Jin et al. "Pinusolide Isolated from Biota orientalis Inhibits 5-Lipoxygenase Dependent Leukotriene C4 Generation by Blocking c-Jun N-Terminal Kinase Pathway in Mast Cells"<br>*Biological & Pharmaceutical Bulletin*, Vol. 35, No. 8, 31 August 2012 (2012-08-31), pages 1374-1378<br>    page 1374, right column | 1-19 |

Form PCT/ISA/210 (second sheet) (July 2022)

**INTERNATIONAL SEARCH REPORT**
Information on patent family members

International application No.

**PCT/CN2023/127216**

| Patent document cited in search report | | | Publication date (day/month/year) | Patent family member(s) | | | Publication date (day/month/year) |
|---|---|---|---|---|---|---|---|
| CN | 114028281 | A | 11 February 2022 | None | | | |
| CN | 114948940 | A | 30 August 2022 | CN | 114948940 | B | 09 May 2023 |
| KR | 100739225 | B1 | 13 July 2007 | None | | | |
| CN | 110478296 | A | 22 November 2019 | None | | | |
| CN | 102885928 | A | 23 January 2013 | CN | 102885928 | B | 23 July 2014 |
| KR | 20030052689 | A | 27 June 2003 | KR | 100444102 | B1 | 11 August 2004 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310403390